# EUROPÄISCHE PATENTSCHRIFT

(11) **EP 1 438 436 B1**
(45) Veröffentlichungstag und Bekanntmachung des Hinweises auf die Patenterteilung: **20.01.2010**
(21) Anmeldenummer: 02802273.9
(22) Anmeldetag: 25.10.2002
(51) Int. Cl.: C12Q 1/68

(54) **VERFAHREN ZUR ANALYSE GENOMISCHER METHYLIERUNGSMUSTER**
METHOD FOR ANALYSIS OF GENOMIC METHYLATION MODELS
PROCEDE D'ANALYSE D'UN MODELE DE METHYLATION GENOMIQUE

(30) Priorität: 26.10.2001 DE 10154318
(43) Veröffentlichungstag der Anmeldung: 21.07.2004
(73) Patentinhaber: Epigenomics AG, 10178 Berlin (DE)
(72) Erfinder: BERLIN, Kurt, 14532 Stahnsdorf (DE); ROY, Debjani, 10115 Berlin (DE)
(74) Vertreter: Schubert, Klemens
(86) Internationale Anmeldenummer: PCT/DE2002/004053
(87) Internationale Veröffentlichungsnummer: WO 2003/038120

(56) Entgegenhaltungen:
- WO-A-00/36141
- WO-A-01/62961
- US-A- 5 871 921
- US-B1- 6 210 884

## Beschreibung

### Gebiet der Erfindung

Die Erfindung betrifft ein Verfahren zur Analyse von Methylierungsmustern in genomischer DNA. Die Erfindung offenbart ein Verfahren zur Analyse methylierter Cytosine. Dies ist von besonderem Interesse, da aberrierende Cytosin-Methylierungsmuster in genomischer DNA mit einer Vielzahl von Krankheiten in Verbindung gebracht wurden.

### Stand der Technik

Die häufigste kovalente Modifikation genomischer DNA ist die Methylierung von Cytosin zu 5-Methylcytosin. Die Cytosin-Methylierung spielt in der Genexpression und -regulation eine wichtige Rolle und es wurde gezeigt, dass sie entscheidend für den Erhalt normaler zellulärer Funktionen ist. Sie steht in Zusammenhang mit dem genetischen Imprinting, der embryonalen Entwicklung und einer großen Anzahl von Krankheiten, einschließlich Krebs.

Zum Beispiel ist aberrierende DNA-Methylierung innerhalb von CpG-Inseln bei menschlicher Malignität, die zur Aufhebung oder Überexpression eines breiten Spektrums von Genen führt, weit verbreitet (P. A. Jones, Cancer Res 65:2463-2467, 1996). Es wurde auch gezeigt, dass abnorme Methylierung in CpG-reichen regulatorischen Elementen in intronischen und kodierenden Teilen von Genen für bestimmte Tumore auftreten (M. F. Chan et al., Curr Top Microbiol Immunol 249:75-86, 2000). Unter Verwendung von "Restriction Landmark Genomic Scanning" waren Costello und Mitarbeiter in der Lage zu zeigen, dass Methylierungsmuster Tumor-Typ-spezifisch sind (J. F. Costello et al., Nat Genet 24:132-138, 2000). Höchst charakteristische DNA-Methylierungsmuster konnten auch für Brustkrebs-Zell-Linien nachgewiesen werden (T. H.-M. Huang et al., Hum Mol Genet 8:459-470, 1999). Die Genom-weite Erfassung des Methylierungsstatus stellt einen molekularen Fingerabdruck von Krebsgewebe dar.

Die Identifizierung von 5-Methylcytosin als ein Bestandteil der genetischen Information ist deshalb von beträchtlichem Interesse. 5-Methylcytosin-Positionen können jedoch nicht durch Sequenzierung identifiziert werden, da 5-Methylcytosin die gleiche Basenpaarungsbildung wie Cytosin hat. Weiterhin geht die vom 5-Methylcytosin getragene epigenetische Information während der PCR-Amlifikation vollständig verloren.

Gegenwärtig basiert das am häufigsten verwendete Analyseverfahren für DNA auf 5-Methylcytosin auf der Reaktion von Bisulfit mit Cytosin, welches durch nachfolgende alkalische Hydrolyse zu Uracil umgesetzt wird, das in seinem Basenpaarungsverhalten dem von Thymidin entspricht. 5-Methylcytosin bleibt unter diesen Bedingungen jedoch unverändert.

Folglich wird die ursprüngliche DNA in solcher Art umgewandelt, dass das Methylcytosin, welches ursprünglich vom Cytosin durch sein Basenpaarungsverhalten nicht zu unterscheiden war, jetzt durch "normale" molekularbiologische Techniken als einziges verbliebenes Cytosin, beispielsweise durch Amplifikation und Hybridisierung oder Sequenzierung, nachgewiesen werden kann. Insbesondere bevorzugt ist die Amplifikation behandelter Sequenz, gefolgt von der Analyse der CG- und TG-Dinukleotide innerhalb der Amplifikate.

Die Bisulfit-Technik wird bisher bis auf wenige Ausnahmen (z. B. M. Zeschnigk, C. Lich, K. Buiting, W. Doerfler, B. Horstehmke. A single-tube PCR test for the diagnosis of Angelman and Prader-Willi syndrome based on allelic methylation differences at the SNRPN locus. Eur J Hum Genet. 1997 Mär-Apr;5(2):94-8), nur in der Forschung angewendet. Immer aber werden nur kurze, spezifische Fragmente eines bekannten Gens nach einer Bisulfitbehandlung amplifiziert und entweder vollständig sequenziert (A. O-lek, J. Walter, The pre-implantation ontogeny of the H19 methylation imprint. Nat Genet. 1997 Nov.; 17(3):275-6) oder einzelne Cytosin-Positionen durch eine Primer-Extensions-Reaktion (M. L. Gonzalgo, P. A. Jones. Rapid quantitation of methylation differences at specific sites using methylation-sensitive single nucleotide primer extension (Ms-SNuPE). Nucleic Acids Res. 1997 Jun. 15;25(12):2529-31; WO Patent 9500669) oder durch enzymatischen Verdau (Z. Xiong, P. W. Laird. COBRA: a sensitive and quantitative DNA methylation assay. Nucleic Acids Res. 1997 Jun. 15;25(12):2532-4) detektiert. Zusätzlich ist auch die Detektion durch Hybridisierung beschrieben worden (Olek et al.; WO 99 28498).

Im Vergleich zur Sequenzierung ist die Bisulfit-Analyse ein präziseres Verfahren zur Quantifizierung des Methylierungsgrads eines speziellen Targets. Diese Technik ist jedoch aufgrund der Schwierigkeit, die gleichzeitige Analyse multipler CpG-Targets innerhalb einer Target-Nukleinsäure, zum Beispiel durch Multiplex PCR, auszuführen, begrenzt.

Verfahren zur Amplifikation spezifischer DNA-Targets basieren auf Templat-gestützter Primer-Extension durch Polymerasen. Das von diesen Verfahren am häufigsten eingesetzte ist die Polymerase-Kettenreaktion "PCR" (K. Mullis et al., Cold Spring Harbor Symp. Quant. Biol. 51:263-273 (1986): H. Erlich et al., EP 50,424; EP 84,796, EP 258017, EP 237,362; K. Mullis, EP 201,184; K. Mullis et al., U.S. Pat. Nr. 4,683,202; H. Erlich, U.S. Pat. Nr. 4,582,788; R. Saiki et al., U.S. Pat. Nr. 4,683,194 und R. Higuchi "PCR Technology", H. Ehrlich (ed.), Stockton Press, NY, 1989, Seiten 61-68).

Bei der Polymerase-Kettenreaktion folgen auf sukzessive Denaturierungszyklen Annealing und Polymerisation. Im ersten Schritt wird die DNA-Doppelhelix·durch kurzzeitiges Erhitzen denaturiert. Dem folgt das Annealing zweier Primer-Spezies, jede an einen DNA-Strang. Nachfolgend werden die reassoziierten Primer unter Verwendung einer Polymerase verlängert. Darauf folgt die Denaturierung der resultierenden doppelsträngigen Nukleinsäuren, wobei es jedem Strang ermöglicht wird, als Templat für einen anderen Zyklus der Templat-gestützten Primer-Extension zu dienen.

Obwohl sie häufig verwendet wird und hochempfindlich ist, ist ein Hauptnachteil der PCR die Schwierigkeit, multiple Fragmente unter Verwendung multipler Primer (Multiplex PCR) gleichzeitig quantifizierbar zu amplifizieren. Zu diesem Problem tragen eine Anzahl von Faktoren bei, hauptsächlich diese:
1. Die Primer-Wirksamkeit einer jeden Primer-Spezies ist voraussichtlich verschieden, zum Teil ist das den Stabilitäts- und strukturellen Unterschieden zwischen den Primern zuzuschreiben.
2. Die Denaturierungsgeschwindigkeit eines jeden Primer-Targets innerhalb der Templat-Nukleinsäure kann sich unterscheiden, deshalb wird sich auch die Primer-Annealing-Geschwindigkeit zwischen jeder Seite unterscheiden.
3. Aufgrund der exponentiellen Natur der PCR wird jeder leichte Unterschied in der Ausbeute zwischen den Primer-Spezies mit jedem Zyklus amplifiziert.

Eine Alternative zur PCR ist die isotherme DNA-Replikation, insbesondere die Rolling-Circle-Replikation, die auf einem natürlich vorkommenden bakteriellen Verfahren der DNA-Replikation basiert.

In einem solchen System wird eine zirkularisierte Nukleinsäure, bekannt als Amplifikations-Target-Ring (ATC) isotherm unter Verwendung von Rolling-Circle-Replikations-Primern und einer Polymerase repliziert. Es gibt dabei keine Denaturierungs- und Annealingstadien, deshalb ist die DNA-Replikation sowohl kontinuierlich als auch isotherm. Die resultierende DNA umfasst eine verkette lineare DNA mit zum ATC identischer Sequenz.

Es sind verschiedene Varianten des Verfahrens beschrieben worden. U.S. Pat. Nr. 5,871,921 (Landegren et al.) beschreibt ein Verfahren, in welchem die Rolling-Circle-Amplifikation zur Detektion genomischer Varianten verwendet werden kann. In dem Assay wird eine detektierbare Nukleinsäure-Sonde an ein einzelsträngiges Nukleinsäure-Target hybridisiert. Die Hybridisierung der Sonde an die Nukleinsäure findet nur statt, wenn die Target-Sequenz anwesend ist. Die Enden der hybridisierten Sonde werden dann kovalent verbunden, um eine geschlossene Schleife von Sonden-Nukleinsäure zu bilden. Dem folgt das Entfernen nicht zirkularisierter Sonden-Moleküle, z. B. durch Exonuklease-Verdau. Das Target-Molekül kann dann durch Bestimmung des Vorliegens der Verflechtung der verketteten Sonde detektiert werden.

Ein alternatives Verfahren zur Verwendung des Rolling-Circle-Amplifikationprozesses wird in U.S. Pat. Nr. 5,648,245, Fire et al., offenbart. Diese Referenz beschreibt einen 4₋Stufen-Prozess zur Entwicklung einer Koncatemer-Bibliothek. In diesem Verfahren ist der erste Schritt, einen Amplifikations-Target-Ring durch Annealing der Enden einer "Padlock-Sonde" an eine Target-Nukleinsäuresequenz zu bilden, gefolgt von der Ligation der Enden der Padlock-Sonde, zur Bildung einer geschlossenen Schleife. Wenn der Target-Ring gebildet ist, ist der zweite Schritt, eine einzelsträngige Tandem-Sequenz-DNA durch Rolling-Circle-Amplifikation des Amplifikations-Target-Rings herzustellen. Der dritte Schritt erfordert die Umwandlung der einzelsträngigen Tandem-Sequenz-DNA in doppelsträngige Tandem-Sequenz-DNA. Abschließend wird die doppelsträngige Tandem-Sequenz-DNA kloniert oder für In-Vitro-Selektion verwendet.

Das U.S. Pat. Nr. 5,866,377 verwendet Rolling-Circle-Amplifikation als Verfahren, um Varianten in einer Nukleinsäuresequenz zu detektieren. In diesem Verfahren hybridisiert eine Padlock-Sonde derart an eine einzelsträngige Nukleinsäure, dass die Enden aneinander angrenzen. In Anwesenheit einer spezifisch veränderlichen Base am Lokus in der Nähe der Endbase eines der Sonden-Enden wird dann ein Ligationsschritt derart durchgeführt, dass eine Ligase im Stande ist, die Enden des Sondenmoleküls miteinander zu verbinden, um ein zirkularisiertes Molekül zu bilden. Die Detektion der Anwesenheit der verketteten Sonde auf der Target-Nukleinsäure zeigt die Anwesenheit der spezifischen Variante an.

Das U.S. Pat. Nr. 5,854,033 (Lizardi) beschreibt ein ähnliches Assay, bei dem eine verkettete Sonde verwendet wird, um durch Rolling-Circle-Amplifi kation Tandem-Sequenz-DNA herzustellen. Die Tandem-Sequenz wird detektiert, um die Menge der vorliegenden Target-Sequenz zu bestimmen.

Ein Überblick über weitere bekannte Verfahren zur Detektion von 5-Methylcytosin kann aus dem folgenden Übersichtsartikel entnommen werden: T. Rein, M. L. DePamphilis, H. Zorbas, Nucleic Acids Res. 1998, 26, 2255.

Weitere Veröffentlichungen, die sich mit der Verwendung der Bisulfit-Technik zur Detektion von Methylierung in einzelnen Genen beschäftigen, sind: G. Grigg, S. Clark. Sequencing 5-methylcytosine residues in genomic DNA. Bioessays. 1994 Jun; 16(6):431-6; M. Zeschnigk, B. Schmitz, B. Dittrich, K. Buiting, B. Horstehmke, W. Doerfler. Imprinted segments in the human genome: different DNA methylation patterns in the Prader-Willi/Angelman syndrome region as determined by the genomic sequencing method. Hum Mol Genet. 1997 März;6(3):387-95; R. Feil, J. Charlton, A. P. Bird, J. Walter, W. Reik. Methylation analysis on individual chromosomes: improved protocol for bisulphite genomic sequencing. Nucleic Acids Res. 1994 Feb 25;22(4):695-6; V. Martin, S. Ribieras, X. Song-Wang, M. C. Rio, R. Dante. Genomic sequencing indicates a correlation between DNA hypomethylation in the 5' region of the pS2 gene and its expression in human breast cancer cell lines. Gene. 1995 Mai 19;157(1-2):261-4;' WO 97 46705, WO 95 15373 und WO 45560.

Eine Übersicht über den Stand der Technik in der Oligomer-Array-Herstellung lässt sich aus einer im Januar 1999 erschienenen Sonderausgabe von Nature Genetics (Nature Genetics Supplement, Volume 21, January 1999) und der dort zitierten Literatur entnehmen.

Fluorophor-markierte Sonden-Oligonukleotide werden häufig zum Scannen immobilisierter DNA-Arrays verwendet. Die einfache Anlagerung von Cy3- und Cy5-Farbstoffen an das 5'-OH der spezifischen Sonde ist besonders für Fluoreszenzmarker geeignet. Die Detektion der Fluoreszenz der hybridisierten Sonden kann beispielsweise via ein konfokales Mikroskop erfolgen. Cy3- und Cy5-Farbstoffe sind, neben vielen anderen, kommerziell erhältlich.

Zusätzlich zur einfachen Detektion der Fluoreszenz-Marker werden häufig zwei weitere Detektionsverfahren in der Nukleinsäure-Analyse verwendet, die Wechselwirkung zwischen zwei Molekülen, bei welcher der angeregte Zustand des einen Moleküls (der Donor) Energie auf das andere Molekül (der Akzeptor) überträgt.Das Donor-Molekül ist ein Fluorophor während das Akzeptor-Molekül einer sein kann aber nicht sein muss. Der Energietransfer geschieht ohne die Emission eines Photons und basiert auf den Dipol-Dipol-Wechselwirkungen zwischen beiden Molekülen. Moleküle, die gewöhnlich bei FRET verwendet werden, schließen Fluoreszin, N,N,N',N'-Tetramethyl-6-carboxyrhodamin (TAMRA), 6-Carboxy-X-rhodamin (ROX), 4-(4'-Dimethylaminophenylazo)benzoesäure (DABCYL) und 5-(2'-Aminoethyl)aminonaphthalin-1-sulfonsäure (EDANS) ein.

Ein weiteres Detektionsverfahren zur Analyse von Biomolekülen, das fluoreszierende Moleküle verwendet, ist die Anwendung von Fluoreszenzpolarisation. Fluoreszenzpolarisation beruht darauf, dass linear polarisiertes Licht die Eigenschaft hat, von stationären fluoreszierenden Molekülen emittiert zu werden. Wenn linear polarisiertes Licht verwendet wird, um ein fluoreszierendes Molekül anzuregen, wird das Molekül zwischen Anregung und Emission nur dann linear polarisiertes Licht emittieren, wenn es stationär ist. Größere Moleküle, d. h. solche mit größerem Molekulargewicht und/oder Volumen, drehen sich langsamer um ihre Achsen als kleinere Moleküle. Die Anwendung von FP-Techniken in der Nukleinsäure-Analyse ist in der Patentanmeldung EP 0382433 B1 offenbart.

Matrix-unterstützte Laserdesorptions/Ionisations-Massenspektrometrie (MALDI-TOF) ist eine sehr wirksame Entwicklung zur Analyse von Biomolekülen (M. Karas, F. Hillenkamp. Laser desorption ionization of proteins with molecular mässes exeeding 10,000 daltons. Anal Chem. 1988 Okt 15;60(20):2299-301). Ein Analyt wird in eine lichtabsorbierende Matrix eingebettet. Die Matrix wird durch einen kurzen Laserpuls verdampft, wodurch das Analytmolekül in unfragmentiertem Zustand in die Dampfphase überführt wird. Der Analyt wird durch Zusammenstoss mit Matrix-Molekülen ionisiert. Eine angelegte Spannung beschleunigt die Ionen in eine feldfreie Driftröhre. Aufgrund ihrer unterschiedlichen Massen werden die Ionen auf unterschiedliche Geschwindigkeiten beschleunigt. Kleinere Ionen erreichen den Detektor schneller als größere.

MALDI-TOF-Spektrometrie ist hervorragend zur Analyse von Peptiden und Proteinen geeignet. Die Analyse von Nukleinsäuren ist um einiges schwieriger (I. G. Gut, S. Beck. DNA and Matrix Assisted Laser Desorptions Ionization Mass Spectrometry. Current Innovations and Future Trends. 1995, 1;147-57). Die Empfindlichkeit für Nukleinsäuren ist ungefähr 100 mal schlechter als für Peptide und sinkt disproportional mit steigender Fragmentgröße. Bei Nukleinsäuren, die ein mehrfach negativ geladenes Rückgrat haben, ist der Ionisationsprozess über die Matrix wesentlich weniger effektiv. In der MALDI-TOF-Spektrometrie spielt die Auswahl der Matrix eine ganz besonders wichtige Rolle. Für die Desorption von Peptiden sind verschiedene sehr wirkungsvolle Matrizes gefunden worden, die eine sehr feine Kristallisation erzeugen. Es gibt jetzt verschiedene entsprechende Matrizes für DNA, jedoch ist der Unterschied in der Empfindlichkeit nicht verringert worden. Der Unterschied in der Empfindlichkeit kann durch solche chemische Modifizierung der DNA verringert werden, bei denen sie einem Peptid ähnlicher wird. Phosphorthioat-Nukleinsäuren, bei denen die üblichen Phosphate des Rückgrats durch Thiophosphate ersetzt werden, können unter Verwendung einfacher Alkylierungsverfahren (I. G. Gut, S. Beck. A procedure for selective DNA alkylation and detection by mass spectrometry. Nucleic Acids Res. 1995 Apr 25;23(8):1367-73) in eine ladungsneutrale DNA überführt werden. Die Kopplung eines Ladungs-Markers an diese modifizierte DNA resultiert in einem Anstieg der Empfindlichkeit auf das gleiche Niveau, das man für Peptide ermittelt hat. Ein weiterer Vorteil des Ladungs-Taggings ist die gesteigerte Stabilität der Analyse gegenüber Verunreinigungen, welche die Detektion unmodifizierter Substrate wesentlich erschweren.

Genomische DNA wird aus DNA aus Zellen, Gewebe oder anderen Test-Proben unter Verwendung von Standardverfahren erhalten. Diese Standard-Methodik findet sich in Referenzen wie Fritsch und Maniatis Ed., Molecular Cloning: A Laboratory Manual, 1989.

Des Weiteren wird in der WO 01/62961 A1 (Epigenomics AG) ein Verfahren zum Nachweis von 5-Methylcytosin in genomischen DNA-Proben beschrieben.

### Beschreibung

Die Erfindung offenbart ein Verfahren zur Analyse der Methylierung von CpG-Dinukleotiden in genomischer DNA. Sie bietet Vorteile gegenüber dem Stand der Technik, indem sie eine quantitative Analyse des Methylierungsgrades an einer spezifischen CpG-Stelle oder multiplen Stellen in einer DNA-Probe erlaubt. Das ist besonders dann entscheidend, wenn die Analyse des Methylierungszustandes die Amplifikation multipler DNA-Fragmente in einer Probe erforderlich macht, welche unter Verwendung von Multiplex-PCR nicht quantifizierbar amplifiziert werden können.

Allgemein umfasst das Verfahren die folgenden Schritte. Zuerst wird die genomische DNA aus zellulären oder anderen Quellen erhalten. Die interessierende DNA (die Target-DNA) wird dann aus der genomischen DNA isoliert. Im nächsten Schritt kann die isolierte DNA einer methylierungsspezifischen Behandlung, wie einer Bisulfitbehandlung oder dem Verdau mit methylierungssensitiven Restriktionsenzymen (der mit den vorhergehenden Schritt kombiniert werden kann), unterworfen werden, was zur Bildung einer einzelsträngigen DNA führt, gefolgt von der Amplifikation eines der beiden Stränge. Dem folgt ein Zirkulisierungs-Schritt zur Ausbildung eines Amplifikations-Target-Rings, der dann in einer Replikationsreaktion unter Verwendung von Rolling-Cycle-Replikations-Primern und einer Polymerase transkribiert wird. Die replizierte Nukleinsäure nimmt die Form einer linearen Nukleinsäure an, welche verkettet Tandem-Kopien der Amplifikations-Target-Ring-Sequenz umfasst.

Im ersten Schritt des Verfahrens wird die DNA aus zellulären, gewebs oder anderen Quellen unter Verwenudng von Standardmethoden erhalten, wie in Referenzen wie Fritsch und Maniatis eds. Molecular Cloning: A Laboratory Manual, 1989, beschrieben. Die extrahierte DNA kann dann unter Verwendung von bekannten Standardmethoden fragmentiert werden, wie beispielsweise, ohne darauf beschränkt zu sein, durch Verdau mit Restriktionsendonukleasep

Der zweite Schritt des Verfahrens ist eine methylierungsspezifische Behandlung des genomischen DNA-Fragments, die zur Bildung von einzelsträngigen Nukleinsäuren führt. In einer Ausführungsform des Verfahrens kann die Behandlung unter Verwendung methylierungssensitiver Endonukleasen durchgeführt werden. Für einen Fachmann wird es offensichtlich sein, dass ein Verdau mit methylierungssensitiver Endonuklease in einer solchen Art durchgeführt werden kann, die geeignet ist, zwischen methylierten und unmethylierten CpG-Dinukleotiden zu unterscheiden, wie es durch J. F. Costello et al., Nat Genet 24:132-138, 2000, beschrieben wurde. Der Verdau kann so durchgeführt werden, dass die CpG-Inseln innerhalb der Sequenz, beispielsweise durch Verwendung eines Restriktionsenzyms, das für die Sequenz TTAA spezifisch ist, intakt bleiben. Alternativ kann der Verdau unter Verwendung von Restriktionsenzymen durchgeführt werden, die innerhalb der CpG-Inseln verdauen und damit die Analyse des Methylierungsstatus erlauben. In einer weiteren bevorzugten Ausführungsform kann dieser Schritt mit dem vorhergehenden Schritt, in welchem die interessierende DNA durch den Verdau mit methylierungssensitiven Restriktionsenzymen isoliert wird, kombiniert werden.

Dem methylierungssensitiven Verdau folgt dann die Amplifikation des interessierenden Strangs, beispielsweise unter Anwendung asymmetrischer PCR. Die resultierenden einzelsträngigen Fragmente werden dann im dritten Schritt des Verfahrens mittels eines Ligations-Oligonukleotids in eine zirkuläre Konformation ligiert.

In einer alternativen Ausführungsform kann der zweite Schritt die Form einer chemischen Behandlung annehmen. Die genomischen DNA-Fragmente können so behandelt werden, dass Cytosin-Basen in der DNA, die nicht in der 5-Position methyliert sind, in eine Base überführt werden, die ungleiche Basenpaarungseigenschaften besitzt. Cytosine, die in der 5-Position methyliert sind, verbleiben durch diese Behandlung jedoch unverändert. In einer bevorzugten Ausführungsform wird die Behandlung unter Verwendung eines Bisulfit-Reagenzes (z. B. Hydrogensulfit, Disulfit) durchgeführt. An den nicht-methylierten Cytosin-Basen findet eine Addition statt. Weiterhin ist es erforderlich, dass ein Denaturierungsreagenz oder Lösemittel wie auch ein Radikalfänger anwesend sind. Eine nachfolgende alkalische Hydrolyse resultiert in der Umsetzung von nicht-methylierten Cytosin-Nukleobasen in Uracil. Die chemisch umgewandelte einzelsträngige DNA kann dann zur Detektion der methylierten Cytosine verwendet werden.

Da jedes CG-Dinukleotid, abhängig von seinem Methylierungsstatus, zu einem TG-Dinukleotid umgesetzt werden oder unumgesetzt bleiben kann, hat Bisulfit-behandelte, CpG-reiche DNA verschiedene einzigartige Eigenschaften. Zuerst werden die Sense- oder Antisense-Stränge derart umgewandelt, dass sie nicht länger komplementär sind. Zusätzlich führt die Bisulfit-Behandlung CpG-reicher DNA zur Erzeugung zweier Strang-Spezies, einer ist relativ Thiamidin-reich und der andere relativ Cytosin-reich. Weiterhin kann jede CG-Position in beiden Strängen der DNA analysiert werden, da jedes CG-Dinukleotid innerhalb des Sense-Strangs an ein CG-Dinukleotid des Antisense-Strangs der DNA hybridisiert.

In einer alternativen Ausführungsform des Verfahrens kann der Behandlungsschritt des Verfahrens einen Verdau mit einem methylierungssensitiven Restriktionsenzym, gefolgt von einem Bisulfit-Behandlungsschritt, umfassen.

Im dritten Schritt des Verfahrens wird die behandelte, einzelsträngige DNA mittels eines Oligonukleotids in eine zirkuläre Konformation ligiert. Zuerst wird die DNA mit einem speziell konstruierten Oligonukleotid, bekannt als Ligations-Oligonukleotid, in Kontakt gebracht, was zur Bildung einer zirkulären Hybrid-Spezies der DNA führt, welche nachfolgend ligiert wird, um eine kovalent geschlossene zirkuläre DNA zu bilden.

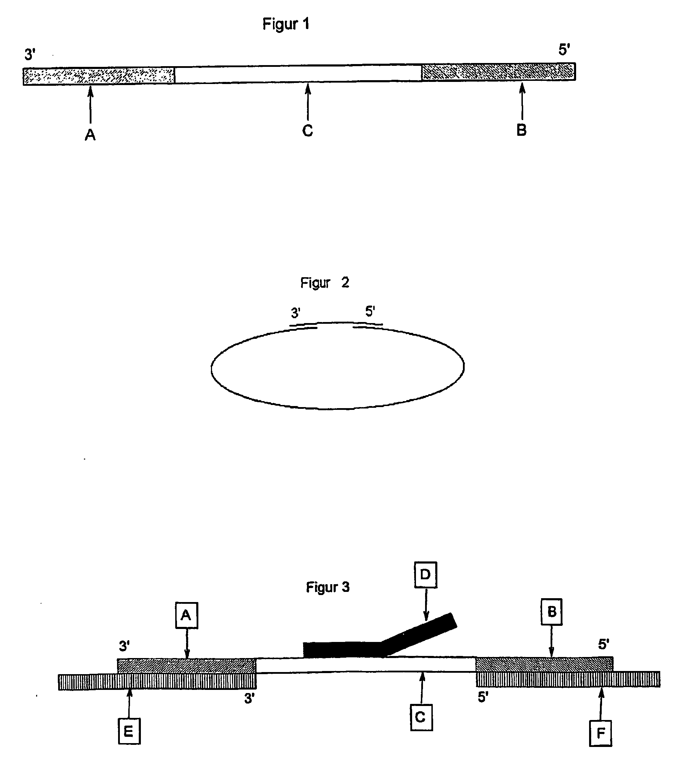

Das Ligations-Oligonukleotid (veranschaulicht in Figur 1) ist ein lineares, einzelsträngiges DNA-Molekül mit einer 3'-Hydroxylgruppe und einer 5'-Phosphatgruppe, was es den Enden erlaubt unter Verwendung einer DNA-Ligase zu ligieren. Erfindungsgemäß sollten die Ligations-Oligonukleotide eine 3'-Hydroxylgruppe, eine linke Target-Sonden-Sequenz, eine Spacer-Sequenz, eine rechte Target-Sonden-Sequenz und eine 5'-Hydroxylgruppe umfassen. Die Spacer-Sequenz sollte eine Primer-Komplementregion zur Bindung von Rolling-Circle-Replikationsprimern einschließen. Die Sequenz der Spacer-Region sollte derart sein, dass sie nicht merklich komplementär zu irgendeiner anderen Sequenz innerhalb der Target-DNA ist.

Die Hybridisierung der Target-Sonden-Regionen an das einzelsträngige Target-DNA-Molekül wird durchgeführt, wobei die Base der linken Target-Sonden-Region mit dem 5'-Ende des DNA-Fragments ein Paar bildet und die Base der rechten Target-Sonden-Region mit dem 3'-Ende des DNA-Fragements ein Paar bildet, was zur Ausbildung einer zirkulären Hybrid-Nukleinsäure führt.

Im vierten Schritt des Verfahrens wird die zirkularisierte Nukleinsäure dann, unter Bedingungen, die zu einer Ligation führen, mit einer Ligase in Kontakt gebracht, so dass die hybridisierten Sektionen des Ligations-Oligonukleotids und der Target-Nukleinsäure ligiert werden, um eine geschlossene zirkuläre Nukleinsäure (nachfolgend Amplifikations-Sonden-Ring oder ATC genannt) zu bilden, was in Figur 2 veranschaulicht wird.

Die zirkularisierte DNA-Fragmente können dann von den linearen Ligationsprodukten durch jegliche im Stand der Technik bekannten Mittel, beispielsweise Gelelektrophorese und Exonuklease-Verdau, isoliert werden, ohne jedoch auf diese beschränkt zu sein.

Im fünften Schritt des Verfahrens wird der ATC unter Verwendung einer Rolling-Circle-Technik repliziert.

Zuerst wird ein Rolling-Circle-Replikations-Primer, unter Bedingungen, die zum Annealing des Primers an den ATC führen, mit dem ATC in Kontakt gebracht. Der Primer sollte aus einer Sequenz bestehen, die zur Primer-KomplementRegion komplementär ist. Die Sequenz des Primers sollte derart sein, dass sie nicht merklich komplementär zu irgendeiner anderen Sequenz innerhalb der zirkularisierten DNA ist. Es ist bevorzugt, dass die bei der Rolling-Circle-Replikation verwendeten Primer am 5'-Ende eine zusätzliche Sequenz enthalten, die nicht komplementär zu irgendeiner Sequenz innerhalb der zirkularisierten DNA ist. Diese Sequenz erleichtert die Ablösung des DNA-Stranges vom ATC.

Dem Annealing des Primers an den ATC (veranschaulicht in Figur 3) nachfolgend, wird die ATC-Sequenz in eine geschlossene lineare Nukleinsäure repliziert, die Tandem-Repeats der ATC-Sequenz (nachfolgend als Tandem-Sequenz-DNA bezeichnet) umfasst. Das wird durch die Addition einer DNA-Polymerase und Nukleotiden erreicht. Von DNA-Polymerase, die für das offenbarte Verfahren geeignet ist, wird verlangt, dass sie zur Rolling-Circle-Replikation Primer-gestützter einzelsträngiger zirkulärer DNA fähig ist. Solche Polymerasen werden nachfolgend als Rolling-Circle-Polymerasen bezeichnet. Es ist bevorzugt, dass die Rolling-Circle-Polymerasen keine 5'- bis 3'-Exonuklease-Aktivität haben und dass die Polymerasen zur Ablösung vom synthetisierten Strang fähig sind. Beispiele geeigneter Polymerasen schließen das Klenow-Fragment der DNA-Polymerase I, Phage M2 DNA-Polymerase, T4 DNA-Polymerase und Bakteriophage .0/.29 DNA-Polymerase ein.

Die Nukleotide, welche in die Tandem-Sequenz-DNA inkorporiert werden, können markiert sein, was die Detektion der Marker im folgenden Schritt erlaubt. Geeignete Marker werden unten beschrieben.

In einer weiteren bevorzugten Ausführungsform der Erfindung werden während des Replikationsschritts des Verfahrens Strang-Ablösungs-Faktoren hinzugefügt. Solche Faktoren schließen DNA-Helicasen wie Kalbsthymus-Helicase oder Proteine wie Einzelstrang-DNA-bindende Proteine und Adenovirus-DNA-bindende Proteine ein.

Im letzten Schritt des Verfahrens wird die Tandem-Sequenz-DNA detektiert

In einer Ausführungsform des Verfahrens können die Nukleotide, die während der ATC-Replikation inkorporiert wurden, mit einem detektierbaren Marker markiert sein, was die Detektion der innerhalb der Tandem-Sequenz-DNA inkorporierten Nukleotide erlaubt. Eine große Auswahl von Molekülen ist zur Verwendung mit dieser Technik geeignet, beispielsweise, ohne auf diese beschränkt zu sein, Massen-, Fluorophor- und radioaktive Marker.

In einer weiteren Ausführungsform der Erfindung kann die Detektion unter Verwendung markierter Sonden-Oligonukleotide durchgeführt werden. Die Sonden Oligonukleotide zeigen durch Hybridisierung an die Tandem-Sequenz-DNA die Anwesenheit spezifischer Sequenzen innerhalb der Tandem-Sequenz-DNA an. Solche Oligonukleotide werden nachfolgend als Detektions-Oligonukleotide bezeichnet. Die detektierbaren Marker können beispielsweise, ohne auf diese beschränkt zu sein, Massen-, Fluoreszenz- (einschließlich FRET und Fluoreszenzpolarisation) und radioaktive Marker einschließen.

In einer weiteren Ausführungsform des Verfahrens können die Rolling-Circle-Replikations-Primer oder Oligonukleotid-Sonden auf einer Festphasen-Oberfläche immobilisiert sein, wobei alle nachfolgenden Schritte des Verfahrens auf der Festphasen-Oberfläche ausgeführt werden. Die Festphasen-Oberfläche besteht bevorzugt aus Silkon, Glas, Polystyrol, Aluminium, Stahl, Eisen, Kupfer, Nickel, Silber oder Gold. Jedoch sind Nitrocellulose wie auch Kunststoffe wie Nylon, welche in Form von Pellets oder auch als Harz-Matrizen vorliegen können, genauso möglich.

Weiterhin können multiple Primer auf der Festphasen-Oberfläche in Form eines Arrays immobilisiert sein, was die Analyse hoher Durchsatzmengen multipler DNA-Proben erlaubt.

Deshalb ist ein weiterer Gegenstand der Erfindung ein Verfahren zur Herstellung eines an einem Trägermaterial fixierten Arrays, zur Analyse des Methylierungsstatus beiner genomischen DNA, wobei in diesem Verfahren mindestens ein erfindungsgemäßes Oligomer an eine feste Phase gekoppelt ist. Verfahren zur Herstellung solcher Arrays mittels Festphasen-Chemie und photolabiler Schutzgruppen sind beispielsweise aus US Patent 5,744,305 bekannt.

Überdies ist ein Gegenstand der Erfindung ein Kit, das beispielsweise aus einem Bisulfit-enthaltenden Reagenz, einem Satz Rolling-Circle-Primer-Oligonukleotiden, Oligonukleotiden und/oder PNA-Oligomeren wie auch einer Anleitung zur Durchführung und Bewertung des beschriebenen Verfahrens, bestehen kann. Ein erfindungsgemäßes Kit kann jedoch auch nur einen Teil der vorher genannten Komponenten enthalten.

In einer weiteren Ausführungsform des Verfahrens kann die CpG-Methylierungs-Analyse auch unter Verwendung eines speziell konstruierten Sonden-Moleküls ausgeführt werden.

Zuerst wird genomische DNA aus zellulären, Gewebe oder anderen Quellen unter Verwendung von Standardverfahren, wie sie sich in Referenzen wie Fritsch und Maniatis Ed., Molecular Cloning: A laboratory Manual, 1989, finden lassen, erhalten. Die extrahierte DNA kann dann unter Anwendung im Stand der Technik bekannter Mittel wie Restriktionsendonukleasen-Verdau, ohne auf diesen beschränkt zu sein, fragmentiert werden.

Im ersten Schritt des Verfahrens wird die DNA so behandelt, dass Cytosin-Basen in der DNA, welche nicht in der 5-Position methyliert sind, in eine Base überführt werden, die ungleiche Basenpaarungseigenschaften hat. Cytosine, die in der 5-Position methyliert sind, verbleiben durch die Behandlung jedoch unverändert. In einer bevorzugten Ausführungsform wird die Behandlung unter Verwendung eines Bisulfit-Reagenzes (z. B. Hydrogensulfit, Disulfit) durchgeführt. An den nicht-methylierten Cytosin-Basen findet eine Addition statt. Weiterhin ist es erforderlich, dass ein Denaturierungsreagenz oder Lösemittel wie auch ein Radikalfänger anwesend sind. Eine nachfolgende alkalische Hydrolyse führt zur Umwandlung von nicht-methylierten Cytosin-Nukleobasen in Uracil. Die chemisch umgewandelte einzelsträngige DNA kann dann zur Detektion der methylierten Cytosine verwendet werden.

Im zweiten Schritt des Verfahrens wird die Target-DNA unter Verwendung einer Nukleinsäure-Sonde, nachfolgend als eine "Open-Circle-Sonde" (OCP) bezeichnet, analysiert. Die OCP wird an die Target-DNA hybridisiert, dann zur Ausbildung einer kovalenten Schleife ligiert, gefolgt von einer Rolling-Circle-Replikation.

Die Sonde entspricht dem vorher beschriebenen Ligations-Oligonukleotid (veranschaulicht in Figur 1), sie ist ein lineares einzelsträngiges DNA-Molekül mit einer 3'-Hydroxylgruppe und einer 5'-Phosphatgruppe, was es den Enden erlaubt unter Verwendung einer DNA-Ligase ligiert zu werden. Die Verwendung der OCPs ist zum Beispiel in U.S. Patent 6,210,884 beschrieben worden. Für das Ziel dieser Erfindung sollte die Sonde eine 3'-Hydroxylgruppe, eine linke Target-Sonden-Sequenz, eine Spacer-Sequenz, eine rechte Target-Sonden-Sequenz und eine 5'-Hydroxylgruppe umfassen. Die Spacer-Sequenz sollte eine Primer-Komplementregion zur Bindung von Rolling-Circle-Replikations-Primern einschließen. Weiterhin kann die Spacer-Region Detektionsmarker- und Promoter-Anteile umfassen. In einer bevorzugten Ausführungsform enthält die OCP keine selbst-komplementären Regionen. Es ist weiterhin bevorzugt, dass innerhalb von OCPs, die einen Promoter-Anteil enthalten, die OCP keine Sequenzen enthält, die einem Transkriptions-Terminator ähneln. Die OCP kann auf einer festen Oberfläche immobilisiert sein, wobei alle weiteren Schritte des Verfahrens auf der Oberfläche durchgeführt werden, was die Analyse hoher Durchsatzmengen von DNA-Proben erlaubt.

### Target-Sonden-Sequenzen

Es gibt zwei Target-Sonden-Sequenzen, eine an jedem Ende der OCP. Die Target-Sonden-Region am 3'-Ende der Sonde wird nachfolgend als linke Target-Sonde bezeichnet, wohingegen die Target-Sonden-Region am 5'-Ende der Sonde nachfolgend als rechte Target-Sonde bezeichnet wird. Jede Target-Sonde ist komplementär zu einer Target-Sequenz innerhalb der behandelten DNA. Die Sequenzen der Target-Sonden sind komplementär zu angrenzenden Target-Regionen der behandelten genomischen DNA. In einer bevorzugten Ausführungsform kann jede Target-Sonde ein CG-Dinukleotid oder mehrere CG-Dinukleotide enthalten

Durch die Hybridisierung der Target-Sonden an die behandelte genomische DNA ist die 3'-Hydroxylgruppe der linken Target-Sonde der 5'-Phosphatgruppe der rechten Target-Sonde benachbart, wodurch beiden Enden erlaubt wird, sich durch eine Ligations-Reaktion miteinander zu verbinden, was zu einer Zirkularisierung der linearen OCP führt.

Die Hybridisierung der Open-Circle-Sonde an die Target-DNA kann so geschehen, dass eine Lücke von einer Base oder mehreren Basen zwischen den 5'- und 3'-Enden der O-pen-Circle-Sonde vorliegt. In einem solchen Fall kann die Lücke vor oder während des Ligationsschritts gefüllt werden. Die Lücke kann durch ein Oligonukleotid oder mehrere Oligonukleotide oder Nukleotide oder eine Kombination beider gefüllt werden. Diese Nukleotide und/oder Oligonukleotide können detektiebare Marker tragen, deren Verwendung später beschrieben wird.

Die ligierte OCP, nachfolgend als Amplifikations-Target-Ring (ATC) bezeichnet, wird dann durch Anwendung der Rolling-Circle-Technik, wie sie vorher beschrieben worden ist, unter Verwendung von Rolling-Circle-Replikations-Primern und Polymerasen repliziert.

### Figuren

### Figur 1

Figur 1 veranschaulicht die Grundstruktur eines Ligations-Oligonukleotids oder einer Open-Circle-Sonde, worin A die linke Target-Sonden-Region ist, B die rechte Target-Sonden-Region ist und C die Spacer-Region ist, welche eine Primer-Komplement-Sequenz einschließt.

### Figur 2

Figur 2 veranschaulicht die Hybridisierung eines Ligations-Oligonukleotids an die Target-Nukleinsäure, woraus die Bildung einer zirkularisierten Nukleinsäure resultiert.

### Figur 3

Figur 3 veranschaulicht das Annealing eines Rolling-Circle-Replikations-Primers an den Amplifikations-Target-Ring. 'A' stellt die linke Target-Sonden-Region des Ligations-Oligonukleotids dar, welche an das 5'-Ende der Target-DNA (E) ligiert ist, 'B' stellt die rechte Target-Sonden-Region des Ligations-Oligonukleotids dar, welche an das 5'-Ende der Target-DNA (F) ligiert ist. C ist der Rolling-Circle-Replikations-Primer, welcher an D, die Spacer-Region des Ligations-Oligonukleotids, anneliert ist.

### Beispiel:

### Assay zum Nachweis des Methylierungsstatus des Gens MDR 1

Im folgenden Beispiel wurde die "rolling-circle"-Amplifikation zur Ermittlung des Methylierungsstatus der CG-Dinukleotide innerhalb der Sequenz CAGGAACAGCGCCGGGGCGTGGGC (SEQ ID NO 1) innerhalb des "Multidrug-Resistenz" Gens mit der Genbank Accessionsnummer NM_000927 (MDR1) verwendet. Im ersten Beispiel (Beispiel 1) wird ein Assay zum Nachweis der Gegenwart von methylierten Versionen der besagten Gene etabliert, im zweiten Beispiel (Beispiel 2) wird ein Assay zum Nachweis der Gegenwart der unmethylierten Version der Gene etabliert. Im dritten Beispiel (Beispiel 3) werden die beiden Assays kombiniert, um den Grad der Methylierung in einer Probe zu bestimmen.

DNA wurde aus Gewebeproben mittels eines Qiagen Extraktionskits gemäß den Spezifikationen des Herstellers extrahiert. Die DNA jeder Probe wurde mit einer Bisulfit (Hydrogensulfit, Disulfit)-Lösung gemäß der "Agarose-Bead"-Methode (Olek et al. 1996) behandelt. Die Behandlung ist derart, dass alle nicht methylierten Cytosine in der Probe in Thiamidin umgewandelt werden, umgekehrt bleibt 5-methyliertes Cytosin unverändert.

### Beispiel 1

### Nachweis der Methylierung innerhalb des "Multidrug-Resistenz" Gens

Das folgende Beispiel beschreibt den Nachweis der Sequenz CAGGAACAGCGCCGGGGCGTGGGC (SEQ ID NO 1) innerhalb des "Multidrug-Resistenz" (MDR1) Gens in seinem CGmethylierten Zustand.

### Hybridisierung und Zirkularisierung des Ligations-Oligonukleotids ("open-circle"-Sonde)

Nach der Isolierung und Bisulfit-Behandlung der genomischen Sequenz mit den oben beschriebenen Mitteln, ist die Sequenz CAGGAACAGCGCCGGGGCGTGGGC (SEQ ID NO 1) in TAGGAATAGCGTCGGGGCGTGGGT (SEQ ID NO 2) umgewandelt worden.
Die folgende Reaktionsmischung wurde dann in einem PCR-Röhrchen zusammen gefügt:
10 µl mit Bisulfit behandelter Target-DNA in 50 mMol Tris-HCl (pH 8,3) wird zu 10 µl einer Ligations-Reaktionsmischung gegeben, enthaltend:
   1,5 U Ampligase thermostabile DNA Ligase (Epicentre Technologies)
   10 mMol MgCl₂
   50 mMol KCl
   0,02% Triton-X-100
   1 mMol Nicotinamid-Adenin-Dinucleotid (NAD) wird hinzu gefügt.
Nach dem Zusatz von 1 µl einer 2 µMol Lösung des Ligations-Oligonukleotids ("open-circle"-Sonde) 5'-Phosphoryl-ACGCTATTCCTATTAGAGACTAGTGTTCTACTAATGTGAATCGATGAGTTAATATTT TACCCACGCCCCG-3'-OH (SEQ ID NO 3) wurde die Ligations-Mischung in einem Eppendorf Thermocycler bei 95°C für 3 Min., gefolgt von 30 Min. bei 65°C und schließlich bei 4 °C für bis zu 5 Stunden bis zur weiteren Verwendung, inkubiert.

### Hybridisierung des Primers und "rolling-circle"-Amplifikation

Zu der Ligations-Mischung wurden 5 µl einer 0,6 µMol Lösung von RCA Primer 5'- CATTAGTAGAACACTAGT -3' (SEQ ID NO 4) gegeben. Nach Inkubation bei 70°C für 5 Min. und Abkühlen auf Raumtemperatur, wurden 5 µl der RCA Reaktion-Mischung enthaltend 800 nMol dNTP's, 50 mMol Tris-HCl, pH 8,3, 25 mMol Ammoniumsulfat und 5 U *Bst* DNA Polymerase large fragment (New England Biolabs), hinzu gegeben und die RCA-Mischung wurde bei 65°C für 1 h inkubiert und bei 4 °C für die weitere Verwendung aufbewahrt.

### Nachweis des RCA-Produkts

Das RCA-Produkt wird auf Polylysin Glasslides (Fischer Scientific) gespottet. Nach dem Trocken und Waschen mit Wasser wird das Slide mit der fluoreszenzmarkierten Oligonukleotid-Sonde Cy5- ACCCACGCCCCGACGCTATTCCTA (SEQ ID NO 5) hybridisiert. Nach dem Auswaschen überschüssiger Sonden wird das Slide in einem Fluoreszenz-Scanner (vom Hersteller Axxon) gescannt. Die Anwesenheit von CG-Methylierung in der Targetsequenz wird durch die Beobachtung einer intensiven Cy5-Fluoreszenz an der entsprechenden Stelle angezeigt.

### Beispiel 2

Nachweis der Sequenz CAGGAACAGCGCCGGGGCGTGGGC (SEQ ID NO 1) innerhalb des "Multidrug-Resistenz" (MDR1) Gens in seinem CG-unmethylierten Zustand.

### Hybridisierung und Zirkularisierung des Ligations-Oligonukleotid ("open-circle"-Sonde)

Nach der Isolierung und Bisulfit-Behandlung der genomischen Sequenz mit den oben beschriebenen Mitteln, ist die Sequenz CAGGAACAGCGCCGGGGCGTGGGC (SEQ ID NO 1) in TAGGAATAGTGTTGGGGTGTGGGT (SEQ ID NO 6)umgewandelt worden. Die folgende Reaktionsmischung wurde dann in einem PCR-Röhrchen zusammen gefügt:
10 µl mit Bisulfit behandelter Target-DNA in 50 mMol Tris-HCl (pH 8,3) wird zu 10 µl einer Ligations-Reaktionsmischung gegeben, enthaltend:
   1,5 U Ampligase thermostabile DNA Ligase (Epicentre Technologies)
   10 mMol MgCl₂
   50 mMol KCl
   0,02% Triton-X-100
   1 mMol Nicotinamid-Adenin-Dinucleotid (NAD) wird hinzu gefügt.
Nach dem Zusatz von 1 µl einer 2 µmol Lösung des Ligations-Oligonukleotids ("open-circle"-Sonde) 5'-Phosphoryl-ACACTATTCCTATTAGAGACTAGTGTTCTACTAATGTGAATCGATGAGTTAATATTT TACCCACACCCCA-3'-OH (SEQ ID NO 7) wurde die Ligations-Mischung in einem Eppendorf Thermocycler bei 95°C für 3 Min., gefolgt von 30 Min. bei 65°C und schließlich bei 4 °C für bis zu 5 Stunden bis zur weiteren Verwendung, inkubiert.

### Hybridisierung des Primers und "rolling-circle"-Amplifikation

Zu der Ligations-Mischung wurden 5 µl einer 0,6 µMol Lösung von RCA Primer 5'-ACTAGTGTTCTACTAATG-3' (SEQ ID NO 8) gegeben. Nach Inkubation bei 70 °C für 5 Min. und Abkühlen auf Raumtemperatur, wurden 5 µl der RCA ReaktionsMischung, enthaltend 800 nMol dNTP's, 50 mMol Tris-HCl, pH 8,3, 25 mMol Ammoniumsulfat und 5 U *Bst* DNA Polymerase large fragment (New England Biolabs), hinzu gegeben und die RCA-Mischung wurde bei 65°C für 1 h inkubiert und bei 4 °C für die weitere Verwendung aufbewahrt.

### Nachweis des RCA-Produkts

Das RCA-Produkt wird auf Polylysin Glasslides (Fischer Scientific) gespottet. Nach dem Trocken und Waschen mit Wasser wird das Slide mit der fluoreszenzmarkierten Oligonukleotid-Sonde Cy3- ACCCACACCCCAACACTATTCCTA (SEQ ID NO 9) hybridisiert. Nach dem Auswaschen überschüssiger Sonden wird das Slide in einem Fluoreszenz-Scanner (vom Hersteller Axxon) gescannt. Die Anwesenheit von CG-Methylierung in der Targetsequenz wird durch die Beobachtung einer intensiven Cy3-Fluoreszenz an der entsprechenden Stelle angezeigt.

### Beispiel 3

Dieses Beispiel beschreibt den Nachweis des relativen Grades der CG-Methylierung innerhalb der Sequenz CAGGAACAGCGCCGGGGCGTGGGC (SEQ ID NO 1) des "Multidrug-Resistenz" (MDR1) Gens.

### Hybridisierung und Zirkularisierung des Ligations-Oligonukleotids ("open-circle"-Sonde)

Nach der Isolierung und Bisulfit-Behandlung der genomischen DNA mit den oben beschriebenen Mitteln, wurde die folgende Reaktion in einem PCR-Röhrchen ausgeführt:
10 µl mit Bisulfit behandelter Target-DNA in 50 mMol Tris-HCl (pH 8,3) wird zu 10 µl einer Ligations-Reaktionsmischung gegeben, enthaltend:
   1,5 U Ampligase thermostabile DNA Ligase (Epicentre Technologies)
   10 mMol MgCl₂
   50 mMol KCl
   0,02% Triton-X-100
   1 mMol Nicotinamid-Adenin-Dinucleotid (NAD) wird hinzu gefügt.
Nach dem Zusatz von 1 µl einer 2 µMol Lösung des Ligations-Oligonukleotids ("open-circle"-Sonde) 5'-Phosphoryl-ACACTATTCCTATTAGAGACTAGTGTTCTACTAATGTGAATCGATGAGTTAATATTT TACCCACACCCCA -3'-OH (SEQ ID NO 7)und 5'-Phosphoryl-ACGCTATTCCTATTAGAGACTAGTGTTCTACTAATGTGAATCGATGAGTTAATATTT TACCCACGCCCCG-3'-OH (SEQ ID NO 3) wurde die Ligations-Mischung in einem Eppendorf Thermocycler bei 95°C für 3 Min., gefolgt von 30 Min. bei 65°C und schließlich bei 4 °C für bis zu 5 Stunden bis zur weiteren Verwendung, inkubiert.

### Hybridisierung des Primers und "rolling-circle"-Amplifikation

Zu der Ligations-Mischung wurden 5 µl einer 0,6 µMol Lösung von RCA Primer 5'-ACTAGTGTTCTACTAATG-3' (SEQ ID NO 8) gegeben. Nach Inkubation bei 70 °C für 5 Min. und Abkühlen auf Raumtemperatur, wurden 5 µl der RCA ReaktionsMischung, enthaltend 800 nMol dNTP's, 50 mMol Tris-HCl, pH 8,3, 25 mMol Ammoniumsulfat und 5 U *Bst* DNA Polymerase large fragment (New England Biolabs), hinzu gegeben und die RCA-Mischung wurde bei 65°C für 1 h inkubiert und bei 4 °C für die weitere Verwendung aufbewahrt.

### Nachweis des RCA-Produkts

Das RCA-Produkt wird auf Polylysin Glasslides (Fischer Scientific) gespottet. Nach dem Trocken und Waschen mit Wasser wird das Slide mit einer equimolaren Mischung der fluoreszenzmarkierten Sonden Cy5-ACCCACGCCCCGACGCTATTCCTA (SEQ ID NO 5) und Cy3-ACCCACACCCCAACACTATTCCTA (SEQ ID NO 9) hybridisiert. Nach dem Auswaschen überschüssiger Sonden wird das Slide in einem Fluoreszenz-Scanner (z. B von Axxon) gescannt. Der Grad der CG-Methylierung in der Targetsequenz wird durch die Beobachtung des Verhältnisses der Cy5/Cy3-Fluoreszenz an der entsprechenden Stelle angezeigt.

## Patentansprüche

1. Verfahren zur Analyse von Cytosin-Methylierung in einer genomischen Probe, die folgenden Schritte umfassend:
a) Isolierung einer Target-DNA aus einer genomischen DNA-Probe
b) Behandlung der DNA in einer Art, die geeignet ist, methylierte von unmethylierten Cytosin-Basen zu unterscheiden
c) in Kontakt bringen der Target-DNA-Sequenz mit einem Ligations-Oligonukleotid unter Bedingungen, die zur Bildung zirkularisierter Hybride der Target-DNA-Ligations-Oligonukleotid-Nukleinsäure führen, wobei
- das Ligations-Oligonukleotid eine einzelsträngige lineare Nukleinsäure umfasst, welche von 5' zu 3' eine 5'-Phosphatgruppe, eine rechte Target-Sonde, eine Spacer-Region, eine linke Target-Sonde und eine 3'-Hydroxylgruppe umfasst, wobei
- die Sequenz der Target-Sonden-Regionen des Ligations-Oligonukleotids mindestens ein CG-Dinukleotid umfasst, und wobei
- das Ligations-Oligonukleotid nur dann an eine einzelsträngige behandelte DNA hybridisiert, wenn die in der Untersuchung befindlichen spezifischen CpG-Dinukleotide in der genomischen DNA in einem spezifischen Methylierungsstatus waren;
d) in Kontakt bringen der zirkularisierten Target-DNA-Ligations-Oligonukleotid-Nukleinsäure mit einer Ligase unter Bedingungen, die zur Bildung einer geschlossenen ringförmig ligierten Nukleinsäure (nachfolgend als ATC bezeichnet) führen
e) in Kontakt bringen des ATC mit einem Primer-Oligonukleotid unter Bedingungen, die zur Hybridisierung führen
f) Hinzufügen von Polymerase zur Templat-DNA unter Bedingungen, welche die Replikation der Templat-DNA fördern, wobei die Replikation der Templat-DNA in der Bildung einer linearen Nukleinsäure resultiert, welche Tandem-Repeats der Templat-DNA-Sequenz umfasst
g) Detektion der Tandem-Sequenz-DNA.

2. Verfahren gemäß Anspruch 1, worin Schritt a) den Restriktions-Endonuklease-Verdau durch mindestens ein Restriktions-Enzym umfasst.

3. Verfahren gemäß Anspruch 1, **dadurch gekennzeichnet, dass** Schritt b) den Verdau mit einem methylierungssensitiven Restriktionsenzym, gefolgt von der Amplifikation von Strang 1 an die Target-DNA, umfasst.

4. Verfahren gemäß Anspruch 1, **dadurch gekennzeichnet, dass** Schritt b) eine derartige Behandlung umfasst, dass die methylierten Cytosin-Basen in der Probe in eine Base umgewandelt werden, die ungleiche Basenpaarungseigenschaften besitzt.

5. Verfahren gemäß Anspruch 1, **dadurch gekennzeichnet, dass** Schritt b) den Verdau mit einem methylierungssensitiven Restriktions-Enzym umfasst, gefolgt von einer derartigen Behandlung, dass methylierte Cytosin-Basen in der Probe in eine Base überführt werden, welche ungleiche Basenpaarungseigenschaften hat.

6. Verfahren gemäß der Ansprüche 4 und 5, worin die Behandlung mittels einer Bisulfit-Lösung durchgeführt wird.

7. Verfahren zur Ermittlung genetischer und/oder epigenetischer Parameter in einer genomischen DNA-Sequenz durch Analyse der Cytosin-Methylierungen, **dadurch gekennzeichnet, dass** die folgenden Schritte ausgeführt werden:
a) Behandlung der DNA in einer Art, die geeignet ist, methylierte von unmethylierten Cytosin-Basen zu unterscheiden, so dass die resultierende Target-DNA einzelsträngig ist;
b) die vorbehandelte einzelsträngige DNA wird mit wenigstens einer Open-Circle-Sonde unter solchen Bedingungen in Kontakt gebracht, die zur Hybridisierung führen, wobei
- die Open-Circle-Sonde eine einzelsträngige lineare Nukleinsäure umfasst, welche von 5' zu 3' eine 5'-Phosphatgruppe, eine rechte Target-Sonde, eine Spacer-Region, eine linke Target-Sonde und eine 3'-Hydroxylgruppe umfasst, wobei
- die Sequenz der Target-Sonden-Regionen der Open-Circle-Sonde mindestens ein CG-Dinukleotid umfasst, und wobei
- die Open-Circle-Sonde nur dann an eine einzelsträngige behandelte DNA hybridisiert, wenn die in der Untersuchung befindlichen spezifischen CpG-Dinukleotide in der genomischen DNA in einem spezifischen Methylierungsstatus waren;
c) an die Target-DNA hybridisierten Open-Circle-Sonden werden ligiert, um Amplifikations-Sonden-Ringe (ATC) zu bilden;
d) ein Primer-Oligonukleotid wird unter solche Bedingungen mit dem Amplifikations-Sonden-Ring in Kontakt gebracht, welche die Primer-Oligonukleotid-ATC-Hybridisierung unterstützen;
e) Polymerase wird unter solchen Bedingungen zur Primer-Oligonukleotid/ATC-Mischung hinzugefügt, welche die Replikation des ATC unterstützen, wobei die Replikation des ATC in der Bildung von Tandem-Sequenz-DNA resultiert;
f) Detektion der Tandem-Sequenz-DNA.

8. Verfahren gemäß Anspruch 7, **dadurch gekennzeichnet, dass** Schritt a) einen Verdau mit methylierungssensitiven Restriktionsenzymen, gefolgt von der Amplifikation eines Stranges der Target-DNA, umfasst.

9. Verfahren gemäß Anspruch 7, **dadurch gekennzeichnet, dass** Schritt a) eine solche Behandlung umfasst, dass die methylierten Cytosin-Basen innerhalb der Probe in Basen umgewandelt werden, die verschiedene Basenpaarungseigenschaften hat.

10. Verfahren gemäß Anspruch 7, **dadurch gekennzeichnet, dass** Schritt a) einen Verdau mit methylierungssensitiven Restriktionsenzymen umfasst, gefolgt von einer solchen Behandung, dass methylierte Cytosin-Basen in der Probe in Basen umgewandelt werden, die andere Basenpaarungseigenschaften hat.

11. Verfahren gemäß der Ansprüche 9 und 10, worin die Behandlung mittels einer Bisulfit-Lösung durchgeführt wird.

12. Verfahren gemäß der Ansprüche 7 bis 11, **dadurch gekennzeichnet, dass** die Open-Circle-Sonde ein CG-Dinukleotid oder mehrere CG-Dinukleotide umfasst.

13. Verfahren gemäß der Ansprüche 7 bis 12, worin durch die Hybridisierung der Open-Circle-Sonde an die vorbehandelte genomische DNA eine Lücke von einer Base oder mehreren Basen zwischen den 5'- und 3'-Enden der Open-Circle-Sonde besteht, die mittels einer oder mehrer Oligonukleotid- oder Nukleotid-Spezies oder deren Kombination gefüllt wird.

14. Verfahren gemäß Anspruch 13, **dadurch gekennzeichnet, dass** die Oligonukleotide und/oder Nukleotide, welche die Lücke füllen, einen detektierbaren Marker tragen.

15. Verfahren gemäß der Ansprüche 1 bis 6, worin das Ligations-Oligonukleotid an einer festen Phase immobilisiert ist.

16. Verfahren gemäß Anspruch 15, worin multiple Ligations-Oligonukleotide an einer festen Phase immobilisiert sind.

17. Verfahren gemäß der Ansprüche 7 bis 14, worin die Open-Circle-Sonde an einer festen Phase immobilisiert ist.

18. Verfahren gemäß Anspruch 17, worin multiple Open-Circle-Sonden an einer festen Phase immobilisiert sind.

19. Verfahren gemäß der Ansprüche 15 bis 18, worin eine oder mehrere Nukleinsäuren an einer festen Phase in Form eines Arrays immobilisiert sind.

20. Verfahren gemäß Anspruch 19, **dadurch gekennzeichnet, dass** die immobilisierten Nukleinsäuren auf einer ebenen festen Phase in Form eines rechtwinkligen oder hexagonalen Gitters angeordnet sind.

21. Verfahren gemäß der Ansprüche 15 bis 20, **dadurch gekennzeichnet, dass** die feste Phase aus Silikon, Glas, Polystyrol, Aluminium, Stahl, Eisen, Kupfer, Nickel, Silber oder Gold besteht.

22. Verfahren, wie in einem der Ansprüche 1 bis 7 beschrieben, worin wenigstens eine Spezies der markierten Nukleotide in die Tandem-Sequenz-DNA inkorporiert wird.

23. Verfahren, wie in einem der Ansprüche 1 bis 7 beschrieben, worin der Detektionsschritt die Hybridisierung eines markierten Detektions-Oligonukleotids oder Peptid-Nukleinsäure-(PNA)-Oligomers umfasst.

24. Verfahren, wie in Anspruch 23 beschrieben, **dadurch gekennzeichnet, dass** die Marker ablösbare Molekül-Fragmente mit einer typische Masse sind, welche im Massenspektrometer detektiert werden.

25. Verfahren, wie in Anspruch 24 beschrieben, **dadurch gekennzeichnet, dass** die Marker im Massenspektrometer detektiert werden.

26. Verfahren, wie in einem der Ansprüche 23 und 24 beschrieben, **dadurch gekennzeichnet, dass** die erzeugten Fragmente eine einfache positive oder negative Nettoladung haben.

27. Verfahren, wie in einem der Ansprüche 24 bis 26 beschrieben, **dadurch gekennzeichnet, dass** die Detektion mittels Matrix-unterstützte Laserdesorptions/Ionisations-Massenspektrometrie (MALDI) oder unter Verwendung von Elektronen-Spray-Massenspektrometrie (ESI) durchgeführt und sichtbar gemacht wird.

28. Verfahren gemäß Anspruch 23, **dadurch gekennzeichnet, dass** die detektierbaren Marker Radionuklide sind.

29. Verfahren gemäß Anspruch 23, **dadurch gekennzeichnet, dass** die Marker Fluorophor-Moleküle sind.

30. Verfahren gemäß Anspruch 29, **dadurch gekennzeichnet, dass** mehr als eine Spezies Fluorophor-Marker verwendet wird.

31. Verfahren gemäß Anspruch 30, worin zwei Typen von Markern verwendet werden, ein Typ ein Donor-Fluorophor und der andere Typ ein Akzeptor-Fluorophor ist und worin der Fluoreszenz-Resonanzenergietransfer (FRET) zwischen den beiden Molekül-Typen beobachtet wird.

32. Verfahren gemäß einem der Ansprüche 29 und 30, **dadurch gekennzeichnet, dass** die FluoreszenzPolarisation des Markers/der Marker gemessen wird.

33. Ein Kit, das ein Bisulfit(= Disulfit, Hydrogensulfit)-Reagenz, Rolling-Circle-Replikations-Primer, Polymerasen wie auch Ligations-Oligonukleotide gemäß Anspruch 1 oder Open-Circle-Sonden gemäß Anspruch 7 umfasst.

## Claims

1. A method for the analysis of cytosine methylation within a genomic sample comprising the following steps:
a) isolating a target DNA from a genomic DNA sample
b) treating the DNA in a manner capable of distinguishing methylated from unmethylated cytosine bases
c) contacting the target DNA sequence with a ligation oligonucleotide under conditions conducive to the formation of circularised target DNA-ligation oligonucleotide nucleic acid hybrids,
- wherein the ligation oligonucleotide comprises a single stranded linear nucleic acid comprising, from 5' to 3', a 5' phosphate group, a right target probe, a spacer region, a left target probe, and a 3' hydroxyl group, wherein
- the sequence of the target probe regions of the ligation oligonucleotide comprises at least one CG dinucleotide, and wherein
- the ligation oligonucleotide hybridizes to a single stranded DNA only if the specific CpG dinucleotides under analysis were in a specific methylation status within the genomic DNA;
d) contacting the circularised target DNA-ligation oligonucleotide nucleic acid with a ligase under conditions conducive to the formation of a continuous circular ligated nucleic acid (hereinafter referred to as ATC)
e) contacting the ATC with a primer oligonucleotide under conditions conducive to hybridisation
f) adding polymerase to the template DNA under conditions that promote the replication of the template DNA, wherein replication of the template DNA results in the formation of a linear nucleic acid comprising tandem repeats of the template DNA sequence
g) detection of the tandem sequence DNA.

2. A method according to claim 1, wherein step a) comprises restriction endonuclease digest by at least one restriction enzyme.

3. A method according to claim 1, **characterized in that** step b) comprises the methylation sensitive restriction enzyme digest followed by the amplification of strand 1 to the target DNA.

4. A method according to claim 1, **characterised in that** step b) comprises a treatment such that methylated cytosine bases within the sample are converted into a base which has dissimilar base pairing properties.

5. A method according to claim 1, **characterised in that** step b) comprises the methylation sensitive restriction enzyme digest followed by a treatment such that methylated cytosine bases within the sample are converted into a base which has dissimilar base pairing properties.

6. A method according to claims 4 and 5, wherein the treatment is carried out by means of a bisulfite solution.

7. A method for ascertaining genetic and/or epigenetic parameters within a genomic DNA sequence by analyzing the cytosine methylations, **characterized in that** the following steps are carried out:
a) treating the DNA in a manner capable of distinguishing methylated from unmethylated cytosine bases such that the resultant target DNA is single stranded;
b) the pretreated single stranded DNA is contacted with at least one open circle probe under conditions conducive to hybridization, wherein
- the open circle probe comprises a single stranded linear nucleic acid comprising, from 5' to 3', a 5' phosphate group, a right target probe, a spacer region, a left target probe, and a 3' hydroxyl group, wherein
- the sequence of the target probe regions of the open circle probe comprises at least one CG dinucleotide, and wherein
- the open circle probe hybridizes to the single stranded treated DNA only if the specific CpG dinucleotides under analysis were in a specific methylation status within the genomic DNA;
c) the open circle probes hybridised to the target DNA are ligated to form amplification probe circles (ATC);
d) a primer oligonucleotide is contacted with the amplification probe circle under conditions that promote primer oligonucleotide ATC hybridisation;
e) polymerase is added to the primer oligonucleotide / ATC mixture under conditions that promote the replication of the ATC, wherein replication of the ATC results in the formation of tandem sequence DNA
f) detection of the tandem sequence DNA.

8. A method according to claim 7, **characterized in that** step a) comprises a methylation sensitive restriction enzyme digest followed by the amplification of one strand of the target DNA.

9. A method according to claim 7, **characterised in that** step a) comprises a treatment such that methylated cytosine bases within the sample are converted into bases which have dissimilar base pairing properties.

10. A method according to claim 7, **characterised in that** step a) comprises a methylation sensitive restriction enzyme digest followed by a treatment such that methylated cytosine bases within the sample are converted into bases which have dissimilar base pairing properties.

11. A method according to claims 9 and 10, wherein the treatment is carried out by means of a bisulfite solution.

12. A method according to claims 7 to 11, **characterised in that** the open circle probe comprises one CG dinucleotide or more CG dinucleotides.

13. A method according to claims 7 to 12, wherein upon hybridisation of the open circle probe to the pretreated genomic DNA a gap of one base or more bases exists between the 5' and 3' ends of the open circle probe, filled in by means of one or more species of oligonucleotides or nucleotides or a combination thereof.

14. A method according to claim 13, **characterised in that** the gap filling oligonucleotides and/or nucleotides carry a detectable label.

15. A method according to claims 1 to 6, wherein the ligation oligonucleotide is immobilised upon a solid phase.

16. A method according to claim 15, wherein multiple ligation oligonucleotides are immobilised upon a solid phase.

17. A method according to claims 7 to 14, wherein the open circle probe is immobilised upon a solid phase.

18. A method according to claim 17, wherein multiple open circle probes are immobilised upon a solid phase.

19. A method according to claims 15 to 18, wherein one nucleic acid is or more nucleic acids are immobilised upon a solid phase in the form of an array.

20. A method according to claim 19, **characterized in that** the immobilised nucleic acids are arranged on a plane solid phase in the form of a rectangular or hexagonal lattice.

21. A method according to claims 15 to 20, **characterized in that** the solid phase is composed of silicone, glass, polystyrene, aluminium, steel, iron, copper, nickel, silver, or gold.

22. A method as recited in one of claims 1 to 7, wherein at least one species of the labelled nucleotides is incorporated into the tandem sequence DNA.

23. A method as recited in one of claims 1 to 7, wherein the detection step comprises the hybridisation of a labelled detection oligonucleotide or peptide nucleic acid (PNA)-oligomer.

24. The method as recited in claim 23, **characterized in that** the labels are detachable molecule fragments having a typical mass which are detected in a mass spectrometer.

25. The method as recited in claim 24, **characterized in that** the labels are detected in a mass spectrometer.

26. The method as recited in one of claims 23 and 24, **characterized in that** the produced fragments have a single positive or negative net charge.

27. The method as recited in one of claims 24 to 26, **characterized in that** the detection is carried out and visualized by means of matrix assisted laser desorption/ionization mass spectrometry (MALDI) or using electron spray mass spectrometry (ESI).

28. A method according to claim 23, **characterized in that** the detectable labels are radionuclides.

29. A method according to claim 23, **characterized in that** the labels are fluorophore molecules.

30. A method according to claim 29, **characterized in that** more than one species of fluorophore labels is used.

31. A method according to claim 30, wherein two types of labels are used, one type being a donor fluorophore and the other type being an acceptor fluorophore, and wherein the fluorescence resonance energy transfer (FRET) between the two types of molecules is monitored.

32. A method according to one of claims 29 and 30, **characterized in that** the fluorescence polarisation of the label / the labels is measured.

33. A kit comprising a bisulfite (= disulfite, hydrogen sulfite) reagent, rolling circle replication primers, polymerases, as well as ligation oligonucleotides according to claim 1 or open circle probes according to claim 7.

## Revendications

1. Procédé pour l'analyse de la méthylation des cytosines dans un échantillon génomique, comprenant les étapes suivantes :
a) l'isolation d'un ADN cible à partir d'un échantillon d'ADN génomique;
b) le traitement de l'ADN d'une manière qui est appropriée pour opérer une distinction entre des bases de cytosines méthylées et des bases de cytosines non méthylées;
c) la mise en contact de la séquence d'ADN cible avec un oligonucléotide de ligature dans des conditions qui donnent lieu à la formation d'hybrides circularisés de l'acide nucléique de l'oligonucléotide de ligature-l'acide nucléique de l'ADN cible,
- l'oligonucléotide de ligature englobant un acide nucléique linéaire simple brin qui comprend, de l'extrémité 5' à l'extrémité 3', un groupe 5-phosphate, une sonde de ciblage droite, une région faisant office d'espaceur, une sonde de ciblage gauche et un groupe 3'-hydroxyle,
- la séquence des régions des sondes de ciblage de l'oligonucléotide de ligature comprend au moins un dinucléotide CG, et
- l'oligonucléotide de ligature ne s'hybride à un ADN traité de type simple brin que lorsque les dinucléotides CpG spécifiques soumis à l'analyse dans l'ADN génomique se trouvent dans un état de méthylation spécifique;
d) la mise en contact de l'acide nucléique circularisé de l'oligonucléotide de ligature/l'ADN cible avec une ligase dans des conditions qui donnent lieu à la formation d'un acide nucléique ligaturé enroulé (que l'on désigne ci-après par l'abréviation ATC);
e) la mise en contact de l'ATC avec une amorce oligonucléotidique dans des conditions qui donnent lieu à une hybridation;
f) l'addition de polymérase à l'ADN matriciel dans des conditions qui favorisent la réplication de l'ADN matriciel, la réplication de l'ADN matriciel donnant lieu à la formation d'un acide nucléique linéaire qui comprend des répétitions en tandem de la séquence de l'ADN matriciel;
g) la détection de l'ADN à séquence tandem.

2. Procédé selon la revendication 1, dans lequel l'étape a) comprend la digestion par endonucléase de restriction via au moins une enzyme de restriction.

3. Procédé selon la revendication 1, **caractérisé en ce que** l'étape b) comprend la digestion avec une enzyme de restriction sensible à la méthylation, suivie de l'amplification du brin 1 sur l'ADN cible.

4. Procédé selon la revendication 1, **caractérisé en ce que** l'étape b) comprend un traitement tel que les bases de cytosines méthylées dans l'échantillon sont transformées en une base qui possède des propriétés inégales d'appariement de bases.

5. Procédé selon la revendication 1, **caractérisé en ce que** l'étape b) comprend la digestion avec une enzyme de restriction sensible à la méthylation, suivie d'un traitement tel que les bases de cytosines méthylées dans l'échantillon sont transformées en une base qui possède des propriétés inégales d'appariement de bases.

6. Procédé selon les revendications 4 et 5, dans lequel le traitement est mis en oeuvre au moyen d'une solution de bisulfite.

7. Procédé pour la détermination de paramètres génétiques et/ou épigénétiques dans une séquence d'ADN génomique par l'analyse des méthylations des cytosines, **caractérisé en ce qu'**on met en oeuvre les étapes suivantes :
a) le traitement de l'ADN d'une manière qui est appropriée pour opérer une distinction entre des bases de cytosines méthylées et des bases de cytosines non méthylées, si bien que l'ADN cible que l'on obtient est un ADN simple brin;
b) la mise en contact de l'ADN prétraité de type simple brin avec au moins une sonde déroulée dans des conditions qui donnent lieu à l'hybridation,
- la sonde déroulée comprenant un acide nucléique linéaire simple brin qui comprend, de l'extrémité 5' à l'extrémité 3', un groupe 5'-phosphate, une sonde de ciblage droite, une région faisant office d'espaceur, une sonde de ciblage gauche et un groupe 3'-hydroxyle,
- la séquence des régions des sondes de ciblage de la sonde déroulée comprend au moins un dinucléotide CG, et
- la sonde déroulée ne s'hybride à un ADN traité de type simple brin que lorsque les dinucléotides CpG spécifiques soumis à l'analyse dans l'ADN génomique se trouvent dans un état de méthylation spécifique;
c) la ligature des sondes hybridées déroulées à l'ADN cible pour former des sondes d'amplification en anneaux (ATC);
d) la mise en contact d'une amorce oligonucléotidique avec la sonde d'amplification en anneau dans des conditions telles que l'on favorise l'hybridation amorce oligonucléotidique-ATC;
e) l'addition de polymérase au mélange amorce oligonucléotidique/ATC dans des conditions qui favorisent la réplication de l'ATC, la réplication de l'ATC donnant lieu à la formation d'un ADN possédant une séquence tandem;
f) la détection de l'ADN à séquence tandem.

8. Procédé selon la revendication 7, dans lequel l'étape a) comprend une digestion avec des enzymes de restriction sensibles à la méthylation, suivie de l'amplification d'un brin de l'ADN cible.

9. Procédé selon la revendication 7, **caractérisé en ce que** l'étape a) comprend un traitement tel que les bases de cytosines méthylées au sein de l'échantillon sont transformées en bases qui possèdent des propriétés différentes d'appariement de bases.

10. Procédé selon la revendication 7, **caractérisé en ce que** l'étape a) comprend une digestion avec des enzymes de restriction sensibles à la méthylation, suivie d'un traitement tel que des bases de cytosines méthylées dans l'échantillon sont transformées en bases qui possèdent des propriétés différentes d'appariement de bases.

11. Procédé selon les revendications 9 et 10, dans lequel le traitement est mis en oeuvre au moyen d'une solution de bisulfite.

12. Procédé selon les revendications 7 à 11, **caractérisé en ce que** la sonde déroulée comprend un dinucléotide CG ou plusieurs dinucléotides CG.

13. Procédé selon les revendications 7 à 12, dans lequel, via l'hybridation de la sonde déroulée à l'ADN génomique prétraité, on obtient une brèche d'une base ou de plusieurs bases entre les extrémités 5' et 3' de la sonde déroulée, qui est remplie au moyen d'une ou de plusieurs espèces d'oligonucléotides ou de nucléotides, ou encore de leur combinaison.

14. Procédé selon la revendication 13, **caractérisé en ce que** les oligonucléotides et/ou les nucléotides, qui remplissent les brèches, portent un marqueur détectable.

15. Procédé selon les revendications 1 à 6, dans lequel l'oligonucléotide de ligature est immobilisé sur une phase solide.

16. Procédé selon la revendication 15, dans lequel plusieurs oligonucléotides de ligature sont immobilisés sur une phase solide.

17. Procédé selon les revendications 7 à 14, dans lequel la sonde déroulée est immobilisée sur une phase solide.

18. Procédé selon la revendication 17, dans lequel plusieurs sondes déroulées sont immobilisées sur une phase solide.

19. Procédé selon les revendications 15 à 18, dans lequel un ou plusieurs acides nucléiques sont immobilisés sur une phase solide sous la forme d'un réseau.

20. Procédé selon la revendication 19, **caractérisé en ce que** les acides nucléiques immobilisés sur une phase solide plane sont disposés sous la forme d'un réseau rectangulaire ou hexagonal.

21. Procédé selon les revendications 15 à 20, **caractérisé en ce que** la phase solide est constituée de silicone, de verre, de polystyrène, d'aluminium, d'acier, de fer, de cuivre, de nickel, d'argent ou d'or.

22. Procédé, tel que décrit dans l'une quelconque des revendications 1 à 7, dans lequel au moins une espèce des nucléotides marqués est incorporée dans l'ADN à séquence tandem.

23. Procédé, tel que décrit dans l'une quelconque des revendications 1 à 7, dans lequel l'étape de détection comprend l'hybridation d'un oligonucléotide de détection marqué ou d'un oligomère peptide-acide nucléique (PNA) marqué.

24. Procédé, tel que décrit à la revendication 23, **caractérisé en ce que** les marqueurs sont des fragments de molécules amovibles possédant une masse typique, qui sont détectés dans le spectromètre de masse.

25. Procédé, tel que décrit à la revendication 24, **caractérisé en ce que** les marqueurs sont détectés dans le spectromètre de masse.

26. Procédé, tel que décrit dans l'une quelconque des revendications 23 et 24, **caractérisé en ce que** les fragments obtenus possèdent une charge nette simple positive ou négative.

27. Procédé, tel que décrite dans l'une quelconque des revendications 24 à 26, **caractérisé en ce que** la détection est mise en oeuvre et est visualisée au moyen d'une spectrométrie de masse couplant une source d'ionisation laser assistée par une matrice (MALDI) ou en utilisant une spectrométrie de masse en mode électrospray (ESI).

28. Procédé selon la revendication 23, **caractérisé en ce que** les marqueurs détectables sont des radionucléides.

29. Procédé selon la revendication 23, **caractérisé en ce que** les marqueurs sont des molécules de fluorophores.

30. Procédé selon la revendication 29, **caractérisé en ce qu'**on utilise plus d'une espèce de marqueur de type fluorophore.

31. Procédé selon la revendication 30, dans lequel on utilise deux types de marqueurs, un type représentant un fluorophore donneur et l'autre type représentant un fluorophore accepteur, et dans lequel on observe le transfert d'énergie par résonance-fluorescence (FRET) entre les deux types de molécules.

32. Procédé selon l'une quelconque des revendications 29 et 30, **caractérisé en ce qu'**on mesure la polarisation de fluorescence du marqueur/des marqueurs.

33. Nécessaire, qui comprend un réactif à base de bisulfite (= disulfite, hydrogénosulfite), une amorce de réplication en forme de cercle roulant, des polymérases, ainsi que des oligonucléotides de ligature selon la revendication 1 ou des sondes déroulées selon la revendication 7.


### Sequenzprotokoll

<110> Epigenomics AG
<120> verfahren zur Analyse genomischer Methylierungsmuster.
<160> 9
<210> 1
   <211> 24
   <212> DNA
   <213> Homo Sapiens
<400> 1
   caggaacagc gccggggcgt gggc 24
<210> 2
   <211> 24
   <212> DNA
   <213> Artificial sequence
<220>
   <223> chemically treated genomic DNA·(Homo sapiens)
<400> 2
   taggaatagc gtcggggcgt gggt 24
<210> 3
   <211> 70
   <212> DNA
   <213> Artificial sequence
<220>
   <223> chemically treated genomic DNA (Homo sapiens)
<400> 3
   acgctattcc tattagagac tagtgttcta ctaatgtgaa tcgatgagtt aatattttac 60
   ccacgccccg 70
<210> 4
   <211> 18
   <212> DNA
   <213> Artificial sequence
<220>
   <223> chemically treated genomic DNA (Homo sapiens)
<400> 4
   cattagtaga acactagt 18
<210> 5
   <211> 24
   <212> DNA
   <213> Artificial Sequence
<220>
   <223> chemically treated genomic DNA (Homo sapiens)
<400> 5
   acccacgccc cgacgctatt ccta 24
<210> 6
   <211> 24
   <212> DNA
   <213> Artificial sequence
<220>
   <223> chemically treated genomic DNA (Homo sapiens)
<400> 6
   taggaatagt gttggggtgt gggt 24
<210> 7
   <211> 70
   <212> DNA
   <213> Artificial sequence
<220>
   <223> chemically treated genomic DNA (Homo sapiens)
<400> 7
   acactattcc tattagagac tagtgttcta ctaatgtgaa tcgatgagtt aatattttac 60
   ccacacccca 70
<210> 8
   <211> 18
   <212> DNA
   <213> Artificial sequence
<220>
   <223> chemically treated genomic DNA (Homo sapiens)
<400> 8
   actagtgttc tactaatg 18
<210> 9
   <211> 24
   <212> DNA
   <213> Artificial Sequence
<220>
   <223> chemically treated genomic DNA (Homo sapiens)
<400> 9
   acccacaccc caacactatt ccta 24